(19) **Europäisches Patentamt / European Patent Office / Office européen des brevets**

(11) **EP 1 574 521 B1**

(12) **EUROPEAN PATENT SPECIFICATION**

(45) Date of publication and mention
of the grant of the patent:
**20.05.2009 Bulletin 2009/21**

(21) Application number: **03780938.1**

(22) Date of filing: **19.12.2003**

(51) Int Cl.:
*C07K 16/00* (2006.01)       *C12P 21/08* (2006.01)
*C07K 1/00* (2006.01)        *C12N 15/09* (2006.01)
*A61K 39/395* (2006.01)

(86) International application number:
**PCT/JP2003/016362**

(87) International publication number:
**WO 2004/056872 (08.07.2004 Gazette 2004/28)**

(54) **METHOD OF PROTECTING OF THIOL GROUPS IN ANTIBODIES**

VERFAHREN ZUM SCHUTZ VON THIOLGRUPPEN IN ANTIKÖRPERN

PROCEDE DE PROTECTION DU GROUPE THIOL D'UN ANTICORPS

(84) Designated Contracting States:
**AT BE BG CH CY CZ DE DK EE ES FI FR GB GR HU IE IT LI LU MC NL PT RO SE SI SK TR**

(30) Priority: **20.12.2002 JP 2002370822**

(43) Date of publication of application:
**14.09.2005 Bulletin 2005/37**

(73) Proprietor: **Mitsubishi Tanabe Pharma Corporation
Chuo-ku
Osaka-shi
Osaka 541-8505 (JP)**

(72) Inventors:
• **SASAKI, Kenji,
MITSUBISHI PHARMA CORPORATION
Chuo-ku,
Tokyo 103-8405 (JP)**
• **KATSUMURA, Yasuhiko
MITSUBISHI PHARMA CORPORATION
Chuo-ku,
Tokyo 103-8405 (JP)**

(74) Representative: **TER MEER - STEINMEISTER & PARTNER GbR
Patentanwälte
Mauerkircherstrasse 45
81679 München (DE)**

(56) References cited:
EP-A- 0 233 044        EP-A- 0 520 499
EP-A- 0 943 690        WO-A-90/13310
WO-A-03/048357         WO-A1-01/75095
WO-A1-97/47735         WO-A1-03/048357
JP-A- 1 231 887        JP-A- 5 304 987
US-B1- 6 342 585

• MURPHY MICHAEL E ET AL: "Protection by glutathione and other thiol compounds against the loss of protein thiols and tocopherol homologs during microsomal lipid peroxidation" EUROPEAN JOURNAL OF BIOCHEMISTRY, vol. 210, no. 1, 1992, pages 139-146, XP002365355 ISSN: 0014-2956
• MORAN LOUISE K ET AL: "Thiols in cellular redox signalling and control" CURRENT MEDICINAL CHEMISTRY, vol. 8, no. 7, June 2001 (2001-06), pages 763-772, XP002365397 ISSN: 0929-8673
• KUCICKI T.J. ET AL.: 'Covalent attachment of sulfhydryl-specific, electron spin resonance spin-labels to fab' fragments of murine monoclonal antibodies that recognize human platelet membrane glycoproteins. Development of membrane protein specific spin probes' BIOCHEMISTRY vol. 25, no. 18, 09 September 1986, pages 4979 - 4983, XP002977504
• SHAHINIAN S. ET AL.: 'A novel strategy affords high-yield coupling of antibody Fab' fragments to liposomes' BIOCHIMICA ET BIOPHYSICA ACTA vol. 1239, no. 2, 1995, pages 157 - 167, XP002974519

SEQUENCE LISTING

<110> Mitsubishi Pharma Corporation

<120> The method of protecting thiol groups in proteins

<130> 03044WO0

<150> JP 2002-370522
<151> 2002-12-20

<160> S

<210> 1
<211> 9
<212> PRT
<213> Homo sapiens

<220>
<223> Inventor: Kenji, Sasaki Yasuhiko,Katsumura

<400> 1

```
        Ile Ser Ser Cys Gly Phe Tyr Trp Asn
        1               5
```

<210> 2
<211> 12
<212> PRT
<213> Homo sapiens

<400> 2

```
        Ile Gly Tyr Ile Tyr Tyr Ser Gly Ser Thr Tyr Tyr
        1               5               10
```

<210> 3
<211> 9
<212> PRT
<213> Homo sapiens

<400> 3

```
Ser Thr Arg Leu Arg Gly Ala Asp Tyr
1               5
```

<210> 4
<211> 17
<212> PRT
<213> Homo sapiens

<400> 4

```
Lys Ser Ser Gln Ser Val Leu Tyr Asn Ser Asn Asn Lys Lys Tyr Leu
1               5                   10                  15
Ala
```

<210> 5
<211> 7
<212> PRT
<213> Homo sapiens

<400> 5

```
Trp Ala Ser Thr Arg Glu Ser
1               5
```

<210> 6
<211> 9
<212> PRT
<213> Homo sapiens

<400> 6

```
Gln Gln Tyr Tyr Ser Thr Pro Trp Thr
1               5
```

<210> 7
<211> 119
<212> PRT
<213> Homo sapiens

<400> 7

```
Gln Val Gln Leu Gln Glu Ser Gly Pro Gly Leu Val Lys Pro Ser Gln
1               5               10              15
Thr Leu Ser Leu Thr Cys Thr Val Ser Gly Gly Ser Ile Ser Ser Cys
            20              25              30
Gly Phe Tyr Trp Asn Trp Ile Arg Gln His Pro Gly Lys Gly Leu Glu
        35              40              45
Trp Ile Gly Tyr Ile Tyr Tyr Ser Gly Ser Thr Tyr Tyr Asn Pro Ser
    50              55              60
Leu Lys Ser Arg Val Thr Ile Ser Leu Asp Thr Ser Lys Ser Gln Phe
65              70              75              80
Ser Leu Lys Leu Ser Ser Leu Thr Ala Ala Asp Thr Ala Val Tyr Tyr
            85              90              95
Cys Ala Arg Ser Thr Arg Leu Arg Gly Ala Asp Tyr Trp Gly Gln Gly
            100             105             110
Thr Met Val Thr Val Ser Ser
        115
```

<210> 8
<211> 114
<212> PRT
<213> Homo sapiens

<400> 8

```
Asp Ile Val Met Thr Gln Ser Pro Asp Ser Leu Ala Val Ser Leu Gly
1               5               10              15
Glu Arg Ala Thr Ile Asn Cys Lys Ser Ser Gln Ser Val Leu Tyr Asn
            20              25              30
Ser Asn Asn Lys Lys Tyr Leu Ala Trp Tyr Gln Gln Lys Pro Gly Gln
        35              40              45
Pro Pro Lys Leu Leu Ile Tyr Trp Ala Ser Thr Arg Glu Ser Gly Val
    50              55              60
Pro Asp Arg Phe Ser Gly Ser Gly Ser Gly Thr Asp Phe Thr Leu Thr
65              70              75              80
Ile Ser Ser Leu Gln Ala Glu Asp Val Ala Val Tyr Tyr Cys Gln Gln
            85              90              95
Tyr Tyr Ser Thr Pro Trp Thr Phe Gly Gln Gly Thr Lys Val Glu Ile
            100             105             110
Lys Arg
```

SEQUENCE LISTING

<110> Mitsubishi Pharma Corporation

<120> The method of protecting thiol groups in proteins

<130> 03044WO0

<150> JP 2002-370822
<151> 2002-12-20

<160> 8

<210> 1
<211> 9
<212> PRT
<213> Homo sapiens

<220>
<223> Inventor: Kenji,Sasaki Yasuhiko,Katsumura

<400> 1

```
Ile Ser Ser Cys Gly Phe Tyr Trp Asn
 1               5
```

<210> 2
<211> 12
<212> PRT
<213> Homo sapiens

<400> 2

```
Ile Gly Tyr Ile Tyr Tyr Ser Gly Ser Thr Tyr Tyr
 1               5                  10
```

<210> 3
<211> 9
<212> PRT
<213> Homo sapiens

<400> 3

```
Ser Thr Arg Leu Arg Gly Ala Asp Tyr
 1               5
```

<210> 4
<211> 17
<212> PRT
<213> Homo sapiens

<400> 4

```
Lys Ser Ser Gln Ser Val Leu Tyr Asn Ser Asn Asn Lys Lys Tyr Leu
 1               5                  10                 15
Ala
```

<210> 5
<211> 7
<212> PRT
<213> Homo sapiens

<400> 5

```
Trp Ala Ser Thr Arg Glu Ser
 1               5
```

<210> 6
<211> 9
<212> PRT
<213> Homo sapiens

<400> 6

Gln Gln Tyr Tyr Ser Thr Pro Trp Thr
1               5

<210> 7
<211> 119
<212> PRT
<213> Homo sapiens

<400> 7

Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Ser Cys
            20                  25                  30
Gly Phe Tyr Trp Asn Trp Ile Arg Gln His Pro Gly Lys Gly Leu Glu
            35                  40                  45
Trp Ile Gly Tyr Ile Tyr Tyr Ser Gly Ser Thr Tyr Tyr Asn Pro Ser
            50                  55                  60
Leu Lys Ser Arg Val Thr Ile Ser Leu Asp Thr Ser Lys Ser Gln Phe
            65                  70                  75              80
Ser Leu Lys Leu Ser Ser Leu Thr Ala Ala Asp Thr Ala Val Tyr Tyr
                    85                  90                  95
Cys Ala Arg Ser Thr Arg Leu Arg Gly Ala Asp Tyr Trp Gly Gln Gly
            100                 105                 110
Thr Met Val Thr Val Ser Ser
            115

<210> 8
<211> 114
<212> PRT
<213> Homo sapiens

<400> 8

Asp Ile Val Met Thr Gln Ser Pro Asp Ser Leu Ala Val Ser Leu Gly
1               5                   10                  15
Glu Arg Ala Thr Ile Asn Cys Lys Ser Ser Gln Ser Val Leu Tyr Asn
            20                  25                  30
Ser Asn Asn Lys Lys Tyr Leu Ala Trp Tyr Gln Gln Lys Pro Gly Gln
            35                  40                  45
Pro Pro Lys Leu Leu Ile Tyr Trp Ala Ser Thr Arg Glu Ser Gly Val
            50                  55                  60
Pro Asp Arg Phe Ser Gly Ser Gly Ser Gly Thr Asp Phe Thr Leu Thr
            65                  70                  75              80
Ile Ser Ser Leu Gln Ala Glu Asp Val Ala Val Tyr Tyr Cys Gln Gln
                    85                  90                  95
Tyr Tyr Ser Thr Pro Trp Thr Phe Gly Gln Gly Thr Lys Val Glu Ile
            100                 105                 110
Lys Arg

**Description**

Technical Field

The present invention relates to a method for efficiently and effectively forming a antibody by protecting a thiol group in a antibody having a free cysteine residue.

Background of the Invention

It is known that when free cysteine residues are present in a protein, a disulfide bond is formed between molecules via thiol groups of the free cysteine residues causing polymerization of the proteins. In addition, it is known also that the thiol group of the free cysteine residue is apt to generate exchange reaction with the disulfide bond formed in a molecule or between molecules, and the free cysteine residues are apt to undergo certain modification.
Since such polymerization, modification or exchange interferes with the formation of a preferable higher-order structure of a protein or generates a change at the active site of the protein, it can become a cause of reduction of activity of the protein.
The above-described reaction by the free cysteine residue frequently occurs at the time of purification or preservation of a protein. In order to prevent such a reaction, a method has been conventionally carried out in which the pH of a protein solution is kept at the acidic condition, or an antioxidant such as cysteine, 2-mercaptoethanol, dithiothreitol, ethylenediaminetetraacetic acid (EDTA), ascorbic acid or the like is added thereto (Seikagaku Jiten (Biochemical Dictionary), 3rd edition, edited by Tokyo Kagaku Dojin, p. 182b).
However, in order to keep the pH of a protein solution at the acidic condition, solvents and buffers to be used in the purification of the protein are limited, so that it is considered that such a limitation results in the reduction of purification efficiency. In addition, the addition of an antioxidant to a solvent or buffer at the time of purification or preservation also has a problem in that sufficient reaction controlling effect cannot be obtained, polymerization cannot be prevented after removal of the antioxidant, or the like.
Accordingly, a method in which the thiol group of a cysteine residue is protected by modifying it with a certain compound can be considered. Examples of the reagent for modifying the thiol group of a cysteine residue of a protein include 1) oxidizing agents such as 5',5-dithiobis(2-nitrobenzoic acid) (DTNB), 2,2'(4,4')-dipyridyl sulfide, tetrathionic acid, 2,6-dichlorophenolindophenol (DCIP) and oxidized glutathione, 2) mercaptide forming agents such as p-mercuribenzoic acid (PMB) and p-mercuribenzenesulfonic acid (PMBS), and 3) alkylating agents such as iodoacetic acid, iodoacetamide and N-ethylmaleimide (NEM) (Seikagaku Jiten (Biochemical Dictionary), 3rd edition, edited by Tokyo Kagaku Dojin, p. 182b). However, since these are used for the purpose of modifying a thiol group of an SH enzyme or the like which requires a thiol group as:an essential factor for its activity, when such a reagent is used with the aim of protecting a free thiol group which is not concerned in the activity of a protein, it conversely results in the obstruction of higher-order structure formation of the protein, thus frequently exerting influence on the activity of protein. On the other hand, even in case that it does not exert influence on the activity of a protein, a problem sometimes occurs when the protein is used as a medicament, such as the necessity to thoroughly remove the remaining reagent. Accordingly, when such a reagent is used for the purpose of protecting free thiol group in a protein, it is preferred to use those which can be used as pharmaceutical additives.
In addition, when a protein to be used for applying to a medicament is produced, it is preferred to use a serum-free culture medium which does not use serum. However, when serum is not used in culturing an animal cell, it frequently results in a case in which supplement of components necessary for maintaining functions of the cell cannot be carried out sufficiently so that a sufficient useful protein cannot be secured, and it also results in a case in which the produced protein is delicately changed physically, biochemically or biologically from the protein of interest, thus posing problems in that, for example, a polymer is apt to be formed and the like. Non-patent Reference:

Seikagaku Jiten (Biochemical Dictionary), 3rd edition, edited by Tokyo Kagaku Dojin, p. 182b

EP-A-0 943 690 relates to a method for industrially producing a human activin A comprising refolding a modified human activin A produced by a microorganism into natural-form human activin A having a biological activity.
WO 90/13310 A relates to the treatment of basic fibroblast growth factor (bFGF) with organic disulfides, preferably glutathione disulfide, or with inorganic compounds of similar function resulting in a bFGF composition of enhanced stability and resistance to multimerization.
US 6.342.585 relates to a method for forming a mixed disulfide of recombinant proteins by solubilization with a denaturing agent in the presence of a disulphide component and then adding a disulfphide component and a denaturing agent at a concentration sufficient to form a mixed disulfide.

Disclosure of the Invention

Objects of the present invention are to establish a method for efficiently and effectively forming a protein by protecting a thiol group in a protein having a free cysteine residue, and to provide a medicament which contains the protein. According to the invention the protein as mentioned throughout the application in an antibody.

Accordingly, the present inventors have accomplished the present invention by carrying out studies on the agents for protecting free cysteine residue and studies on the reaction conditions, and by resolving such problems through in-depth analyses of the reaction products.

That is, the present invention is as defined in claims 1-14.

Brief Description of the Drawings

Fig. 1 is a graph showing the effect to inhibit polymerization of a GAH antibody $F(ab')_2$ by serum-free culturing and the influence of pH.

Fig. 2 is a graph showing the effect to inhibit polymerization of a GAH antibody $F(ab')_2$ by serum-free culturing and the influence of reaction temperature.

Fig. 3 is a graph showing the effect to inhibit polymerization of a GAH antibody $F(ab')_2$ by serum-free culturing and the influence of cystine concentration.

Fig. 4 is a graph showing a cation exchange chromatogram before the activation of a GAH whole antibody.

Fig. 5 is a graph showing a cation exchange chromatogram after the activation of a purified GAH whole antibody.

Fig. 6 is a graph showing a cation exchange chromatogram after the polymerization inhibition treatment of a purified GAH whole antibody.

Fig. 7 is a graph showing a cation exchange chromatogram of purified GAH whole antibody desalted and concentrated after the polymerization inhibition treatment of a purified GAH whole antibody.

Fig. 8 is a graph showing a cation exchange chromatogram of purified GAH antibody $F(ab')_2$.

Best Mode for Carrying Out the Invention

The present invention is described below in detail.

In the present invention, the compounds which have a disulfide bond in the molecule and exert substantially no influence on the activity of the protein are cystine, homocystine, lipoic acid, oxidized glutathione and glutathione disulfide, and cystine is preferred. In addition, a method in which compounds which have thiol groups in the molecules and exert substantially no influence on the activity of the protein are simultaneously or separately added can also be exemplified.

In the present invention, examples of the compound which has a thiol group in the molecule and exerts substantially no influence on the activity of the protein include cysteine, homocysteine, glutathione and dihydrolipoic acid, and cysteine is particularly preferred.

In the present invention, the term "the compound which exerts substantially no influence on the activity of the protein" means that it does not increase or decrease the activity of the protein. As the protein is an antibody, a case in which reactivity of the antigen-antibody reaction does not increase or decrease can be exemplified.

The protein in the present invention is characterized in that it has a free cysteine residue in its molecular structure. The term "free" as used herein means a state in which the cysteine residue has unpaired electron, is unstable and is rich in reactivity. In addition, it is more preferable that the thiol group of the cysteine residue is rich in reactivity.

Regarding the thiol group in the present invention, a case in which it is concerned directly or indirectly in the activity of the protein can be exemplified, and direct concern is more preferred. Examples of the activity of the protein as used herein include the reactivity in an antigen-antibody reaction and the reactivity in an enzyme reaction. Examples of the direct concern include a case in which the activity of the protein is reduced when the thiol group is modified with e.g. a modifying reagent. On the other hand, an example of the indirect concern is a case in which the activity of the protein is reduced due to interrupting of the formation of the higher-order structure by modification of the thiol group.

As the protection in the present invention, inhibition of the polymerization, modification or exchange which exerts substantial influence on the protein activity can be exemplified. More specifically, examples include inhibition of the formation of a polymer due to formation of a disulfide bond between proteins via thiol groups, inhibition of the modification of a free cysteine residue, and inhibition of the exchange reaction with a disulfide bond formed in a molecule or between molecules by the thiol groups of the free cysteine residues. The inhibition of the formation of a polymer due to formation of a disulfide bond between proteins via thiol groups is more preferred. In this case, a thiol-disulfide exchange reaction of the following reaction occurs through the reaction of a compound having a disulfide bond, such as cystine, with the thiol group in a protein having a free cysteine residue.

$$\text{Pr–SH} \quad + \quad \text{R-S-S-R} \quad \rightarrow \quad \text{Pr-S-S-R} \quad + \quad \text{R-SH} \quad (I)$$

(Pr represents a protein and R represents a residue other than functional groups.)

Thus, since the Pr-S-S-R formed by the reaction is stably present even after elimination of the R-S-S-R, the reaction via Pr-SH, such as polymerization of proteins, is inhibited.

In addition, in such a method, the compound which has a disulfide bond in the molecule and exerts substantially no influence on the activity of the protein may be added at any stage at the time of the protein production. For example, when this is carried out using a GAH antibody shown in the following Examples, examples include the methods in which cystine is added to the final purified products of $F(ab')_2$ (Examples 1 to 3) or immediately after the activation of its whole antibody (Examples 4 to 6).

A solvent is used in the methods of the present invention. Although the solvent is not particularly limited, a liquid capable of causing exchange of the thiol group of a cysteine residue in a protein with disulfide of cystine is preferred. Specifically, a buffer of weakly acidic to alkaline, preferably a buffer of neutral to weakly alkaline, is suitable.

The concentration of the adding compound which has a disulfide bond in the molecule and exerts substantially no influence on the activity of the protein is not particularly limited, but in the case of cystine, for example, a cystine concentration capable of causing exchange of the thiol group of cysteine residue of a protein with disulfide of cystine is preferred. Specifically, a concentration of 0.01 to 100 mM, preferably from 0.1 to 10 mM, is suitable.

Although the protein concentration is not particularly limited, a concentration capable of causing exchange of the thiol group of a cysteine residue of a protein with disulfide of a compound which has a disulfide bond in the molecule and exerts substantially no influence on the activity of the protein is preferred. Specifically, a concentration of 0.01 to 1,000 mg/ml, preferably from 0.1 to 100 mg/ml, is suitable.

Although the reaction temperature is not particularly limited, for example, a temperature capable of causing exchange of the thiol group of a cysteine residue of a protein with disulfide of cystine as a compound which has a disulfide bond in the molecule and exerts substantially no influence on the activity of the protein is preferred. Specifically, a temperature of -20 to 60°C, preferably from 0°C to 50°C is suitable.

In addition, even when a compound having a thiol group such as cysteine is present together with a protein in such a method, the reaction of (I) occurs by the addition of excess cystine. For example, as disclosed in WO03/048357, regarding a protein produced using a serum-free medium, there is a case in which the protein having sufficient activity cannot be secured due to insufficient supply of components necessary for maintaining functions of the protein producing cell. In that case, an activation treatment of the protein with cysteine or the like may be carried out, but as shown in the following Examples, polymerization inhibition of GAH antibody can also be carried out immediately after the activation of the whole antibody by cysteine (Example 4).

Examples of the polymerization in the present invention include a change in the higher-order structure of a protein, a change in the activity of the protein, and formation of a dimer, a trimer and the like, which occur when the protein forms an intermolecular disulfide bond via thiol groups. Preferred are a change in the activity of the protein and formation of a dimer and a trimer, and more preferred is formation of a dimer and a trimer.

Examples of the modification in the present invention include a change in the higher-order structure of a protein, a change in the activity of the protein and the like, which occur when a compound capable of exerting substantial influence on the activity is linked to the thiol group in a protein having a free cysteine residue. Preferably, a change in the activity of the protein can be exemplified.

Examples of the exchange in the present invention include a change in the higher-order structure of a protein, a change in the activity of the protein and the like, which occur when the thiol group in a protein having a free cysteine residue forms a thiol-disulfide bond formed in a molecule or between molecules of the protein. Preferably, a change in the activity of the protein can be exemplified.

The recombinant protein in the present invention is an antibody.

Examples of the antibody in the present invention include whole antibodies (full length antibody, whole antibody), antibody fragments (antibody fragments such as $F(ab')$, $F(ab')_2$ and scFv (single chain antibody)) and antibody derivatives, and an $F(ab')_2$ antibody is more preferred.

An example of the monoclonal antibody in the present invention is an antibody which comprises the amino acid sequences represented by SEQ ID NOs:1, 2 and 3 in the Sequence Listing in its heavy chain hypervariable region, and the amino acid sequences represented by SEQ ID NOs:4, 5 and 6 in the Sequence Listing in its light chain hypervariable region. Generally, these amino acid sequences are respectively contained in three hypervariable regions of each chain of the heavy chain and light chain, in order of SEQ ID NOs: 1, 2 and 3 and SEQ ID NOs:4, 5 and 6 starting from the N-terminal side. The hypervariable region determines specificity of immunoglobulin as an antibody and binding affinity of an antigenic determinant with the antibody, and is also called complementarity determining region. Accordingly, the regions other than such hypervariable regions may be derived from other antibody. That is, those in which modification of partial amino acids, such as substitution, insertion, deletion or addition, is carried out within such a range that the binding activity (reactivity) with antigens are not spoiled are included in the monoclonal antibodies which can be used in the present invention.

More specific examples include an antibody which comprises a heavy chain variable region comprising the amino acid sequence represented by SEQ ID NO:7 in the Sequence Listing and a light chain variable region comprising the amino acid sequence represented by SEQ ID NO:8 in the Sequence Listing, namely the GAH antibody. The GAH antibody is a human monoclonal antibody which specifically binds to cancers screened based on the reactivity with gastric cancer and large bowel cancer (EP526700 or EP520499), and this antibody can be obtained by preparing hybridomas of cancer patient-derived lymphocytes with mouse myeloma cells, and selecting a hybridoma having the above-described specified amino acid sequences, or can also be prepared by genetic engineering techniques (EP520499).

When compared with the position of cysteine in conventionally known human amino acid sequences, the GAH antibody is characterized by the 32nd position cysteine in SEQ ID NO:7 in the Sequence Listing. That is, this is considered to be a free cysteine which is not concerned in the disulfide bond formation in the molecule. Since the cysteine residue corresponds to the 4th position in the sequence shown in SEQ ID NO:1 in the Sequence Listing and is positioned in a heavy chain hypervariable region, it is considered that this is concerned in the binding with its antigen.

Examples of the serum-free medium in the present invention include those media which do not contain sera such as (fetal) bovine serum in the medium components. Examples include the serum-free media CD CHO (manufactured by Invitrogen) and ExCell 325-PF (manufactured by JRH) described in the following Examples, and the like.

The present invention provides a medicament which comprises, as an active ingredient, an antibody, obtained by the method of the present invention, for example, a pharmaceutical composition which comprises the above-described substance and a pharmaceutically acceptable carrier, and provides a therapeutic preparation having various dosage forms. The term "pharmaceutically acceptable" means that unpreferable side effects, such as nausea, vertigo and qualm, accompanied by the administration, immune response against a pharmaceutical preparation at the time of its frequent administration, do not occur. In addition, an antibody prepared by binding a substance such as a toxin to the protein of the present invention can also be used as a medicament. Examples include those in which an antibody is bound to liposome into which an agent such as an antitumor agent, e.g., doxorubicin, is encapsulated (EP526700, EP520499 and EP1174126). The antibody-bound anti-neoplastic substance-containing liposome can be made into pharmaceutical preparations by conventionally known methods such as a dehydration method (WO88/06441), a method in which it is used as solutions by adding a stabilizing agent (JP-A-64-9931), a freeze drying method (JP-A-64-9931), and can be administered to patients by a method such as intravascular administration or topical administration. The dose can be optionally selected in response to the kinds of anti-neoplastic substance as the active ingredient, and when a doxorubicin-encapsulated liposome is administered for example, it can be used at a dose of 50 mg/kg or less, preferably 10 mg/kg or less, more preferably 5 mg/kg or less, in terms of the amount of the active ingredient.

Examples

The present invention is described below in detail based on Examples, but the present invention is not limited to the following Examples without overstepping its gist.

Production Example 1

GAH antibody F(ab')$_2$ by serum-free culturing:

(1) Production of whole antibody of GAH antibody (hereinafter referred to as "GAH whole antibody") into medium by serum-free culturing

A cell culture medium was prepared by dissolving 4 mmol of glutamine (manufactured by SIGMA) and 10 mg of insulin (manufactured by SIGMA) in 1 liter of each of serum-free media CD CHO (manufactured by Invitrogen) and ExCell 325-PF (manufactured by JRH) and carrying out aseptic filtration of the solution using a 0.22 $\mu$m bottle top filter (manufactured by Corning Coaster). The thus prepared cell medium was aseptically charged in a 1 liter capacity spinner flask (manufactured by Belco) which had been sterilized in advance using an autoclave (manufactured by Sakura Seiki), and the flask was arranged on a culture controlling device (manufactured by Biott) to adjust the temperature to 37°C, and the dissolved oxygen concentration to 3.0 mg/l, the pH to 7.4 and the agitation speed to 60 rpm.

A recombinant GAH antibody producing CHO cell 1-6R (cf. Examples of EP520499) cultured in advance using a roller bottle (manufactured by Falcon) was treated with trypsin to peel off the cells, the supernatant was discarded after centrifugation, and the cells were washed twice with the corresponding serum-free medium. Thereafter, the cells were suspended in the corresponding serum-free medium and aseptically inoculated into a 1 liter capacity spinner flask to start the culturing. After the inoculation, sampling was carried out and viable cell density and survival ratio were measured using a hemocytometer to find that there respectively CD CHO: $1.09 \times 10^5$ cells/ml and 82.3%, ExCell 325-PF: $0.87 \times 10^5$ cells/ml and 84.1%.

The culturing was completed after 353 hours of the inoculation, and the culture medium was recovered. The culture medium was centrifuged (3,000 rpm, 20 min) and then filtered using a 0.22 μm bottle top filter to obtain about 800 ml of an unpurified bulk.

(2) Purification of GAH whole antibody from unpurified bulk obtained by serum-free culturing

About 800 ml of each of the unpurified bulks obtained in (1) was divided into two and subjected to purification by column chromatography using XK16 column (i.d. 16 mm, manufactured by Amersham Bioscience) packed with 14.3 ml ofProsep-A resin (manufactured by Millipore). The unpurified bulk.and a buffer were fed into the column at a flow rate of 14.3 mL/min, application and washing were carried out by a down-flow mode, and elution and regeneration by an up-flow mode. The buffer composition for washing, elution and regeneration was 40 mM acetate buffer containing 40 mM NaCl having respective pH values of 6.0, 4.0 and 2.7.

From the unpurified bulks of CD CHO and ExCell 325-PF, 47.2 ml and 50.8 ml of whole antibody containing solutions (pH 4.0) were respectively obtained. When amounts of the antibody in these solution were measured by ultraviolet absorption photometry, they were 57 mg and 48 mg, respectively.

(3) Activation treatment of GAH whole antibody

Regarding the activation treatment method, WO03/048357 can be used as a reference.

Using Centricon 30 (manufactured by Amicon), 20 mg of each of the GAH whole antibody solutions obtained in (2) using CD CHO medium and EXCELL 325-PF medium was concentrated to about 1.7 ml. Reagents were added to 250 μl (corresponding to 3 mg) of this liquid to obtain a composition of 30 mM Tris-HCl buffer (pH 9) containing 1.6 M sodium chloride, 2 mM L-cysteine and 12 mM ascorbic acid in final concentrations, and the composition was adjusted to about 10 ml. This liquid was allowed to stand at room temperature for 16 hours and then adjusted to pH 4 by adding trifluoroacetic acid. The liquid was concentrated using Centricon 30, and the liquid composition was replaced by 0.05% acetic acid.

(4) Preparation of F(ab')$_2$ by pepsin treatment of the whole antibody after activation treatment, and its purification

The activation-treated sample obtained in (3) was adjusted to pH 4.0 and then digested with pepsin. That is, pepsin (manufactured by SIGMA) was added thereto to a concentration of 1.2 mg/g GAH, followed by aseptic filtration.using Mylex filter (manufactured by Millipore, 0.22 μm) and then gently stirring for 17 hours under heating at 37°C.

After the pepsin digestion, a GAH antibody F(ab')$_2$ was purified using cation exchange column chromatography. That is, the XK16 column was packed with 15.3 ml of a cation exchange resin SP-Sepharose FF (manufactured by Amersham Bioscience), and the antibody-containing liquid after pepsin digestion was applied thereto. Thereafter, washing was carried out using 40 mM acetate buffer (pH 4.0) containing 20 mM NaCl, and peak of the antibody was fractionated while gradually increasing the salt concentration. The flow rate was 1.58 ml/min. Volumes of the samples of CD CHO and ExCell 325-PF were 10.5 ml and 11.6 ml, respectively.

Example 1

Inhibition of polymerization of GAH antibody F(ab')$_2$ by serum-free culturing (influence of reaction pH):

Inhibition of polymerization was attempted on the GAH antibody F(ab')$_2$ after activation treatment obtained in accordance with Production Example 1 using ExCell 325-PF medium.

A cystine solution was added to a GAH antibody F(ab')$_2$ solution prepared to a concentration of about 5 mg/ml, to a final concentration of 1 mM. In this case, the cystine solution was used by dissolving cystine in 0.5 N hydrochloric acid to a concentration of 40 mM.

This solution was divided into two, 1/4 volume of 1 M sodium phosphate buffer (pH 7.5) was added to one of them (final pH 7.5), nothing was added to the other (final pH 4.7), and they were used as a cystine-added pH 7.5 treated solution and a cystine-added pH 4.7 treated solution, respectively.

These cystine-added solutions and a cystine-un-added solution were allowed to stand at 37°C for 3 hours, and then cystine was removed using an ultrafiltration membrane of nominal molecular weight cutoff of 30 K, and the solvent was replaced by 20 mM acetate buffer (pH 4.7). Using this stage as initial, gel filtration HPLC analysis was carried out. Next, each of these solutions was mixed with 1/4 volume of I M sodium phosphate buffer (pH 7.5) (final pH 7.5), allowed to stand at 37°C for 45 minutes, and then gel filtration HPLC analysis was carried out.

Gel filtration HPLC conditions:

| Detector: | Ultraviolet absorption photometer (measuring wavelength: 215 nm) |
| Column: | TSKgel G3000SW$_{XL}$ (about 8 mm in inner diameter x about 30 cm in length) manufactured by Tosoh |
| Pre-column: | TSKgel guardcolumnSWXL (about 6 mm in inner diameter x about 4 cm in length) manufactured by Tosoh |
| Column temp: | Constant temperature of around 25°C |
| Mobile phase: | 50 mM sodium phosphate buffer (pH 7.0)/300 mM sodium sulfate |
| Flow rate: | 0.3 ml/min |

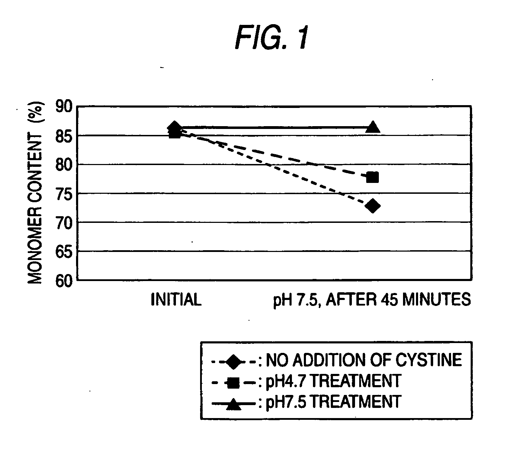

The results are shown in Fig. 1. In the initial, the monomer content was 86 to 87% in each of the cystine-un-added solution, cystine-added pH 4.7 treated solution and cystine-added pH 7.5 treated solution. When these three types of solutions were adjusted to pH 7.5 and allowed to stand at 37°C for 45 minutes, the monomer content of the cystine-un-added solution was reduced to 73%, while that of the cystine-added pH 4.7 treated solution was 78% showing a slight polymerization inhibition. In addition, that of the cystine-added pH 7.5 treated solution was 87%, completely inhibiting the polymerization.

It was found from these results that the reaction pH at the time of the addition of cystine was good at the neutral side than the acidic side.

Example 2

Inhibition of polymerization of GAH antibody F(ab')$_2$ by serum-free culturing (influence of reaction temperature):

Inhibition of polymerization was attempted on the GAH antibody F(ab')$_2$ after activation treatment obtained in accordance with Production Example 1 using ExCell 325-PF medium.

A cystine solution was added to a GAH antibody F(ab')$_2$ solution prepared to a concentration of about 5 mg/ml, to a final concentration of 1 mM. In this case, the cystine solution was used by dissolving cystine in 0.5 N hydrochloric acid to a concentration of 40 mM.

This solution was mixed with 1/4 volume of 1 M sodium phosphate buffer (pH 7.5) (final pH 7.5), divided into two, and one of them was allowed to stand at 37°C for 3 hours, and the other at 4°C for a whole day and night. They were used as a cystine-added 37°C treated solution and a cystine-added 4°C treated solution, respectively

Cystine was removed from these two types of solutions and a cystine-un-added solution using an ultrafiltration membrane of nominal molecular weight cutoff of 30 K, and the solvent was replaced by 20 mM acetate buffer (pH 4.7). Using this stage as initial, gel filtration HPLC analysis was carried out. Next, each of these three types of solutions was mixed with 1/4 volume of 1 M sodium phosphate buffer (pH 7.5) (final pH 7.5), allowed to stand at 37°C for 45 minutes, and then gel filtration HPLC analysis was carried out.

Gel filtration HPLC conditions:

The same as Example 1.

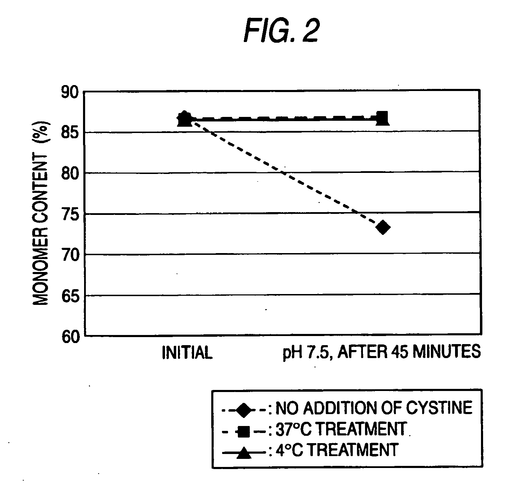

The results are shown in Fig. 2. In the initial, the monomer content was 86 to 87% in each of the cystine-un-added solution, cystine-added 37°C treated: solution and cystine-added 4°C treated solution. When these solutions were adjusted to pH 7.5 and allowed to stand at 37°C for 45 minutes, the monomer content of the cystine-un-added solution was reduced to 73%, while that of the cystine-added 37°C treated solution was 87% and that of the cystine-added 4°C treated solution was 86%, completely inhibiting the polymerization in both cases.

It was found from these results that the reaction temperature at the time of the addition of cystine was either 37°C or 4°C.

Example 3

Inhibition of polymerization of GAH antibody F(ab')$_2$ by serum-free culturing (influence of cystine concentration):

Inhibition of polymerization was attempted on the GAH antibody F(ab')$_2$ after activation treatment obtained in accordance with Production Example 1 using ExCell 325-PF medium.

A cystine solution was added to a GAH antibody F(ab')$_2$ solution prepared to a concentration of about 5 mg/ml, to a final concentration of 1 mM or 0.54 mM In this case, the cystine solution was used by dissolving cystine in 0.5 N hydrochloric acid to a concentration of 40 mM or 20 mM.

Each of these solutions was mixed with 1/4 volume of 1 M sodium phosphate buffer (pH 7.5) (final pH 7.5), and used as a 1 mM treatment and 0.5 mM treatment, respectively.

These two types of solutions and a cystine-un-added solution were allowed to stand at 4°C for a whole day and night, and then cystine was removed using an ultrafiltration membrane of nominal molecular weight cutoff of 30 K, and the solvent was replaced by 20 mM acetate buffer (pH 4.7). Using this stage as initial, gel filtration HPLC analysis was carried out. Next, each of these solutions was mixed with 1/4 volume of 1 M sodium phosphate buffer (pH 7.5) (final pH 7.5), allowed to stand at 37°C for 45 minutes, and then gel filtration HPLC analysis was carried out.

Gel filtration HPLC conditions:

The same as Example 1.

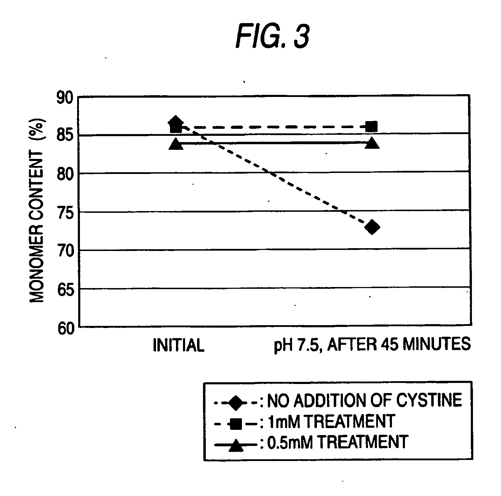

The results are shown in Fig. 3. In the initial, the monomer content was 86 to 87% in each of the cystine-un-added solution and 1 mM cystine-added solution, while that in the 0.5 mM cystine-added solution was a slightly lower value of 84%. When these solutions were adjusted to pH 7.5 and allowed to stand at 37°C for 45 minutes, the monomer content of the cystine-un-added solution was reduced to 73%, while that of the 1 mM cystine-added solution was 86% and that of the 0.5 mM cystine-added solution was 84%, each showing no change from the initial.

It was found from these results that the polymerization inhibitory effect was slightly higher by 1 mM cystine adding concentration than that of 0.5 mM.

Production Example 2

Preparation of GAH whole antibody by serum-free culturing:

(1) Production of GAH whole antibody into medium by serum-free culturing

A cell culture medium was prepared by dissolving a serum-free medium ExCell 325-PF (JRH) in 30 L in total of milli-Q water, 4 mM of glutamine (manufactured by SIGMA), 1.6 g/l of sodium bicarbonate (manufactured by Invitrogen) and 10 mg/l of insulin (manufactured by SIGMA) were dissolved therein and carrying out aseptic filtration of the solution using a 0.22 $\mu$m Milli-pack 40 filter (manufactured by Millipore). In a 50 L capacity fermentor (manufactured by Biott) which had been sterilized in advance using an autoclave (manufactured by Sakura Seiki), 4 L of the thus prepared cell medium was aseptically charged and the vessel was connected to a culture controlling device (manufactured by Biott).

A recombinant GAH antibody producing CHO cell 1-6R (cf. Examples of WO03/048357) was cultured in advance in a 7 L (working volume: 6 L) capacity spinner flask (manufactured by Belco) using a serum-free medium ExCell 325-PF, and the total volume of the culture medium was aseptically inoculated into a 50 L capacity fermentor. Thereafter, the culturing was carried out by supplementing the vessel with 5 L of the serum-free medium ExCell 325-PF after 1 day, 15 L after 2 days and 1 L after 4 days.

The culturing was completed after 307 hours of the inoculation, and the culture medium was recovered. The culture medium was subjected to cell separation by Prostack (manufactured by Millipore) and then filtered using a 0.22 $\mu$m Millipack 40 to obtain about 26 L of an unpurified bulk.

(2) Purification of GAH whole antibody from unpurified bulk obtained by serum-free culturing

A 17 L portion of the unpurified bulks obtained in (1) was divided into 12 portions and subjected to purification by column chromatography using XK16 column (i.d. 16 mm, manufactured by Amersham Bioscience) packed with 14.3 ml of Prosep-A resin (manufactured by Millipore), thereby obtaining about 1.7 g of GAH whole antibody.

Example 4

Activation treatment and polymerization inhibition treatment of GAH whole antibody:

A 640 ml portion of the GAH whole antibody solution obtained in Production Example 2 was diluted with 40 mM acetic acid-sodium acetate solution (pH 4.0) containing 40 mM sodium chloride to an antibody concentration of 1 mg/ml. Reagents were added to this antibody solution to obtain a composition of 30 mM Tris-HCl buffer (pH 9.0) containing 1.0 M sodium chloride, 2 mM L-cysteine and 12 mM ascorbic acid in final concentrations. This liquid was adjusted to pH 7.5 with 5 N sodium hydroxide aqueous solution, and then gently stirred at room temperature for 3 hours to complete the activation reaction. A small portion thereof was sampled, and a reagent was added to the remaining antibody solution to a final cystine concentration of 4 mM. Thereafter, this was adjusted to pH 7.5 with 5 N sodium hydroxide aqueous solution, and then gently stirred at room temperature for 2 hours to carry out the polymerization inhibition treatment. Thereafter, this was adjusted to pH 4.0 with 6 N hydrochloric acid, and desalting and concentration were carried out using an ultrafiltration membrane of nominal molecular weight cutoff of 30 kDa (Hydrosalt, manufactured by Sartorius) until the electric conductivity became 0.6 S/m. The sample taken out after the activation reaction was subjected to desalting and concentration using an ultrafiltration membrane of nominal molecular weight cutoff of 30 kDa (Saltocon, manufactured by Sartorius) without carrying out the polymerization inhibition treatment.

Example 5

Physicochemical analysis of GAH-whole antibody by HPLC:

Using the GAH whole antibody solution obtained in Example 4, cation exchange liquid chromatography was carried out to compare chromatograms before and after the polymerization inhibition treatment.

Operation conditions:

| | |
|---|---|
| Detector: | Ultraviolet absorption photometer (measuring wavelength: 280 nm) |
| Column: | TSKgel CM-5PW (7.5 mm in inner diameter x 7.5 cm in length) manufactured by Tosoh |
| Column temperature: | Constant temperature of around 30°C |
| Mobile phase A | 50 mM sodium phosphate buffer (pH 7:0)w |
| Mobile phase B: | 50 mM sodium phosphate buffer (pH 7.0)/0.5 M sodium chloride |
| Feeding of mobile phases | : Density gradient control (see the following Table 1) |

Table 1

| Time (min) after injection | Mobile phase A (%) | Mobile phase B (%) |
|---|---|---|
| 0 - 40 | 100 → 50 | 0 → 50 |
| 40 - 41 | 50 → 100 | 50 → 0 |

Flow rate:    1.0 ml/min

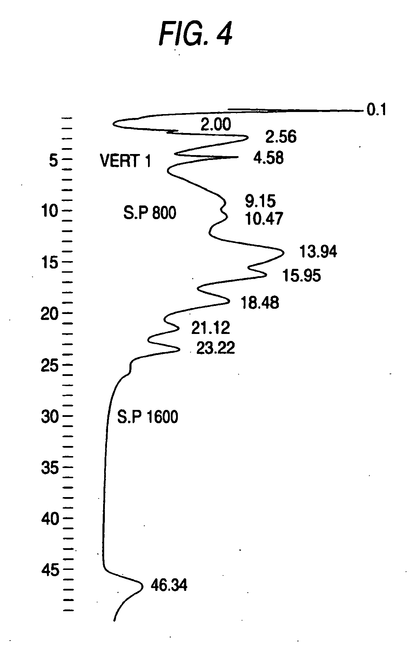

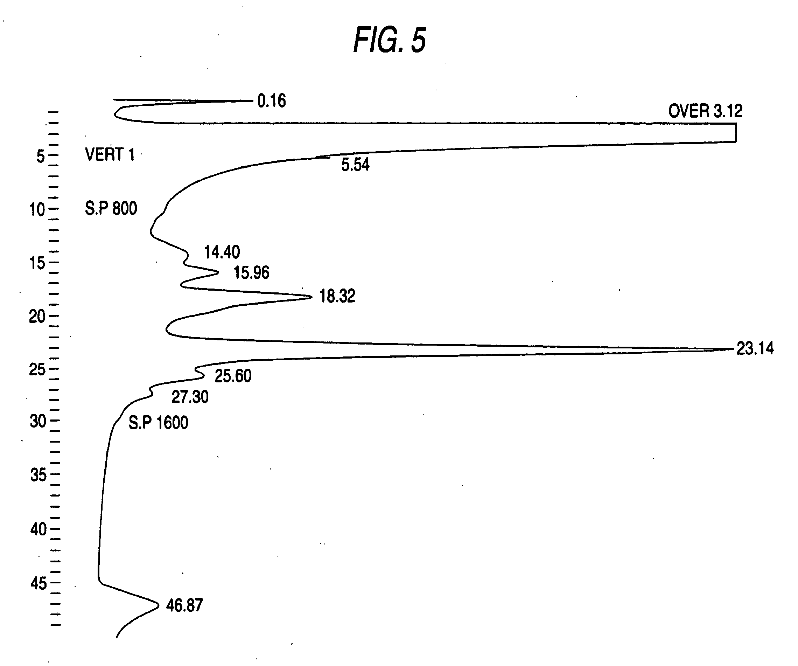

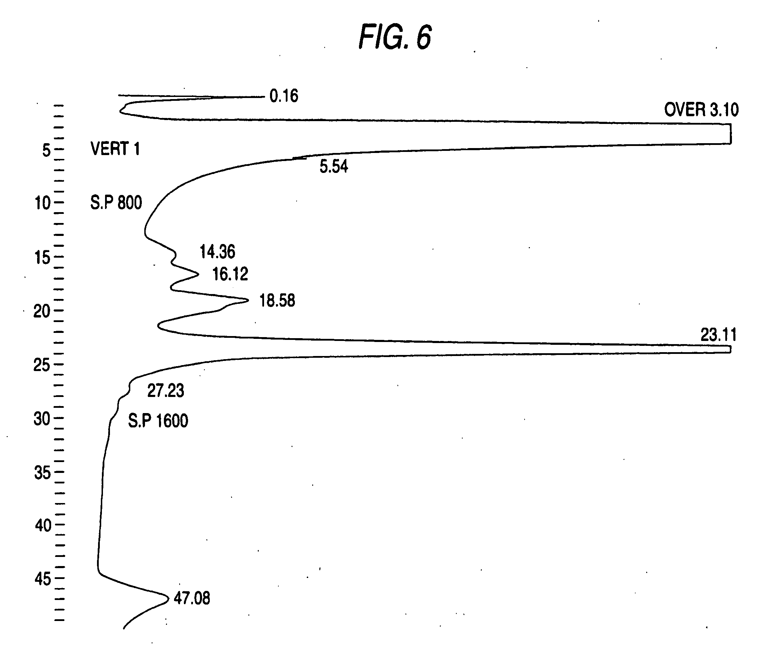

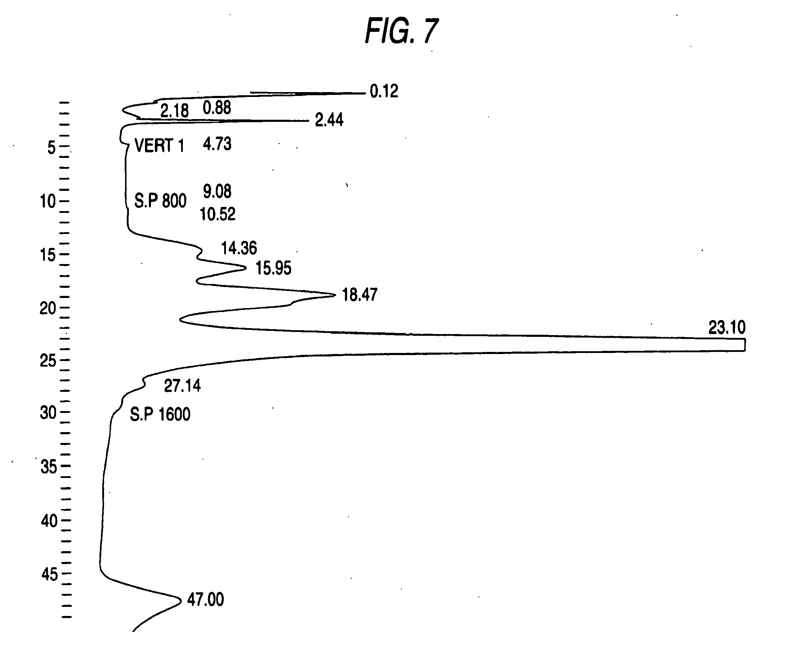

Chromatograms obtained from samples taken off before the activation reaction, after completion of the activation reaction, after the polymerization inhibition treatment and after the desalting and concentration were compared on the purified GAH whole antibody, with the results shown in Fig. 4 to Fig. 7.
The chromatogram before the activation is an inactive type because of the presence of a large number of molecular species originated from the heterogeneity as shown in Fig. 4, but it becomes a chromatogram of active type GAH when the activation reaction is carried out as shown in Fig. 5. Thereafter, differences from the chromatogram of after the activation reaction cannot be found in the chromatogram after carrying out the treatment of the present invention as shown in Fig. 6. In addition, changes cannot be found even when the desalting and concentration treatment is carried out as shown in Fig. 7.
From these results, it can be seen by the comparison of chromatograms that the treatment of the present invention does not exert influence on the activity of GAH antibody.

Example 6

Analysis of polymerization quantity of GAH whole antibody by gel.filtration HPLC

Using the GAH whole antibody solution obtained in Example 4, a gel filtration HPLC analysis was carried out to compare amounts of a monomer of the antibody before and after the polymerization inhibition treatment. The operation conditions are the same as in Example 1.

The results obtained from samples taken off before the activation reaction, after completion of the activation reaction, after the polymerization inhibition treatment and after the desalting and concentration are shown in Table 2, when the treatment of the present invention was applied or not applied to the purified GAH whole antibody.

Table 2

| Comparison of the effect of polymerization inhibition treatment | | | | |
|---|---|---|---|---|
| | Polymerization inhibition treatment | | No polymerization inhibition treatment | |
| | Monomer (%) | Polymer (%) | Monomer (%) | Polymer (%) |
| Before activation reaction | 96.8 | 3.18 | 96.8 | 3.18 |
| After activation reaction | 94.6 | 5.05 | 94.6 | 5.05 |
| After polymerization inhibition treatment | 97.5 | 2.28 | - | - |
| After desalting and concentration | 97.3 | 2.56 | 83.8 | 13.8 |

The purified GAH whole antibody to which the treatment of the present invention was not applied forms a polymer in a large amount at the desalting and concentration step. On the other hand, in the case of the purified GAH whole antibody to which the treatment of the present invention was applied, the monomer content increases after the activation treatment though slight, and polymer is not formed at the desalting and concentration step.

Based on this result, it was found that the treatment of the present invention increases the monomer content and inhibits polymer formation at the same time.

Example 7

Formation of F(ab')$_2$ of the whole antibody by pepsin digestion after its activation reaction and polymerization inhibition treatment (treatment of the present invention), and purification of the same:

A 150 ml portion of a sample was taken off from the solution obtained in Example 4 and digested with pepsin. That is, pepsin (manufactured by SIGMA) was added thereto to a concentration of 1.2 mg/g GAH, followed by aseptic filtration using a bottle top filter (Corning Coaster, 0.22 $\mu$m) and then gently stirring for 19 hours under heating at 37°C.

After the pepsin digestion, the GAH antibody F(ab')$_2$ was purified using cation exchange column chromatography. That is, the XK16 column was packed with 15.3 ml of a cation exchange resin SP-Sepharose HP (manufactured by Amersham Bioscience), and the antibody-containing liquid after pepsin digestion was applied thereto. Thereafter, washing was carried out using 40 mM acetate buffer (pH 4.0) containing 20 mM NaCl, and peak of the antibody was fractionated while gradually increasing the salt concentration. The flow rate was 4.17 ml/min.

Thereafter, the mixture was adjusted to pH 7.0 by carrying out buffer exchange to 5 mM phosphate buffer (pH 4.0) using Saltocon of nominal molecular weight cutoff of 30 kDa, and then the GAH antibody F(ab')$_2$ was purified by anion exchange column chromatography. That is, the XK16 column was packed with 31 ml of an anion exchange resin Q-Sepharose FF (manufactured by Amersham Bioscience), the antibody-containing solution after buffer exchange was applied thereto and peak of the antibody was fractionated. Thereafter, the mixture was adjusted to pH 4.0 and the antibody concentration was adjusted to about 5 mg/ml using Saltocon of nominal molecular weight cutoff of 30-kDa to thereby obtaining a purified GAH antibody F(ab')$_2$.

Example 8

Physicochemical analysis of GAH antibody F(ab')$_2$ by HPLC and measurement of polymer quantity:

Cation exchange liquid chromatography was carried out by applying the GAH antibody F(ab')$_2$ obtained in

Example 7 to TSKgel CM-5PW (7.5 mm in inner diameter, 7.5 cm in length; manufactured by Tosoh). The operation conditions are the same as shown in Example 4.

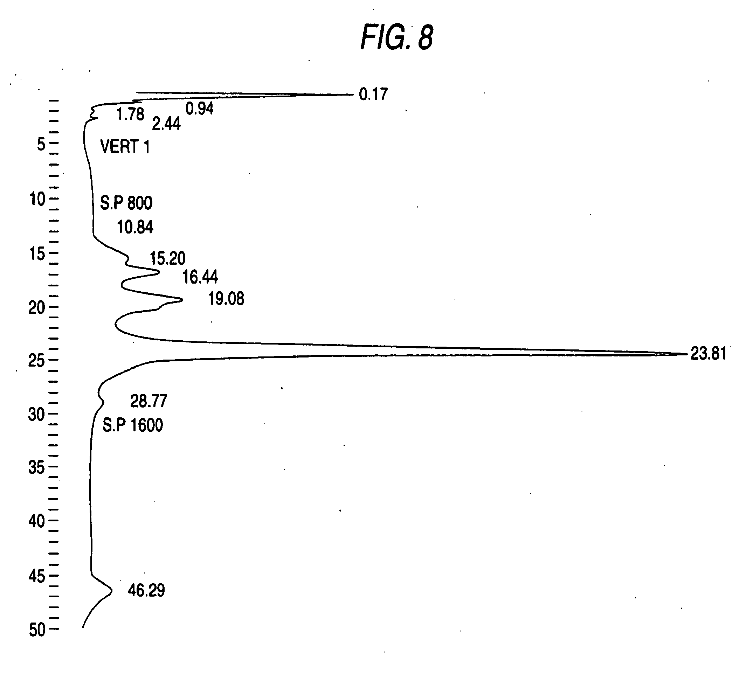

The result is shown in Fig. 8.

It was confirmed by this result that the thus obtained antibody is active type, and the treatment of the present invention does not exert influence on the antibody activity throughout the purification process.

Also, the GAH antibody F(ab')$_2$ obtained in Example 7 was subjected to the measurement of amounts of monomer by using a gel filtration HPLC method.

The operation conditions are the same as shown in Example 1.

As a result, the monomer content was 93.8%, thus revealing that the antibody sufficiently satisfying the purification specification was obtained. In addition, it was confirmed that the treatment of the present invention inhibited formation of polymer throughout the entire purification process, and it was found that sharp improvement of the purification yield was attained.

Industrial Applicability

In the present invention, it is possible to establish a method for efficiently and effectively forming an antibody by protecting a thiol group in an antibody having a free cysteine residue, and to provide a medicament which comprises the antibody.

## Claims

1. A method for protecting a thiol group in an antibody having a free cysteine residue, which comprises adding a compound which has a disulfide bond in the molecule and exerts substantially no influence on the activity of the antibody, wherein the compound which has a disulfide bond in the molecule and exerts substantially no influence on the activity of the antibody is cystine, homocystine, lipoic acid, oxidized glutathione or glutathione disulfide.

**2.** The method according to claim 1 which is for inhibiting a polymerization reaction of antibodies via thiol groups.

**3.** The method according to claim 1 which is for inhibiting modification of an antibody.

**4.** The method according to claim 1 which is for inhibiting an exchange reaction of a thiol group in an antibody with a disulfide bond formed in the molecule or between the molecules of the antibody.

**5.** The method according to any one of claims 1 to 4, wherein the compound which has a disulfide bond in the molecule and exerts substantially no influence on the activity of the antibody is cystine.

**6.** The method for protecting a thiol group in an antibody having a free cysteine residue according to claim 1, which comprises adding the compound which has a disulfide bond in the molecule and exerts substantially no influence on the activity of the antibody simultaneously or separately from a compound which has a thiol group in the molecule and exerts substantially no influence on the activity of the antibody, wherein the compound which has a thiol group in the molecule and exerts substantially no influence on the activity of the antibody is cysteine, homocysteine, glutathione or dihydrolipoic acid.

**7.** The method according to claim 6, wherein the compound which has a thiol group in the molecule and exerts substantially no influence on the activity of the antibody is cysteine.

**8.** The method according to any one of claims 1 to 7. wherein the antibody is an $F(ab')_2$ antibody.

**9.** The method according to any one of claim 1 to 8, wherein the antibody is a monoclonal antibody.

**10.** The method according to claim 9. wherein the monoclonal antibody has a thiol group in its variable region.

**11.** The method according to claim 9 or 10, wherein the monoclonal antibody has a.free cysteine residue in its variable region.

**12.** The method according to any one of claims 9 to 11, wherein the monoclonal antibody comprises the amino acid sequences represented by SEQ ID NOs:1, 2 and 3 in the Sequence Listing in its heavy chain hypervariable region, and the amino acid sequences represented by SEQ ID NOs:4, 5 and 6 in the Sequence Listing in its light chain hypervariable region.

**13.** The method according to any one of claims 9 to 12, wherein the monoclonal antibody comprises a heavy chain variable region comprising the amino acid sequence represented by SEQ ID NO:7 in the Sequence Listing and a light chain variable region containing the amino acid sequence represented by SEQ ID NO:8 in the Sequence Listing.

**14.** The method according to any one of claims 1 to 13, wherein the antibody is produced by using a cell cultured in a serum-free medium.


**Patentansprüche**

**1.** Verfahren zum Schützen einer Thiolgruppe in einem Antikörper mit einem freien Cysteinrest, das das Zusetzen einer Verbindung umfasst, die eine Disulfidbindung im Molekül aufweist und im Wesentlichen keinen Einfluss auf die Aktivität des Antikörpers ausübt, wobei die Verbindung, die eine Disulfidbindung im Molekül aufweist und im Wesentlichen keinen Einfluss auf die Aktivität des Antikörpers ausübt, Cystin, Homocystin, Liponsäure, oxidiertes Glutathion oder Glutathiondisulfid ist.

**2.** Verfahren nach Anspruch 1, das für die Hemmung einer Polymerisationsreaktion von Antikörpern mittels Thiolgruppen ist.

**3.** Verfahren nach Anspruch 1, das für die Hemmung einer Modifizierung eines Antikörpers ist.

**4.** Verfahren nach Anspruch 1, das für die Hemmung einer Austauschreaktion einer Thiolgruppe in einem Antikörper mit einer Disulfidbindung ist, die im Molekül oder zwischen den Molekülen des Antikörpers gebildet wird.

**5.** Verfahren nach einem der Ansprüche 1 bis 4, wobei die Verbindung, die eine Disulfidgruppe im Molekül aufweist und im Wesentlichen keinen Einfluss auf die Aktivität des Antikörpers ausübt, Cystin ist.

**6.** Verfahren zum Schutz einer Thiolgruppe in einem Antikörper mit einem freien Cysteinrest nach Anspruch 1, das das Zusetzen der Verbindung, die eine Disulfidbindung im Molekül aufweist und im Wesentlichen keinen Einfluss auf die Aktivität des Antikörpers ausübt, gleichzeitig mit oder getrennt von einer Verbindung umfasst, die eine Thiolgruppe im Molekül aufweist und im Wesentlichen keinen Einfluss auf die Aktivität des Antikörpers ausübt, wobei die Verbindung, die eine Thiolgruppe im Molekül aufweist und im Wesentlichen keinen Einfluss auf die Aktivität des Antikörpers ausübt, Cystein, Homocystein, Glutathion oder Dihydroliponsäure ist.

**7.** Verfahren nach Anspruch 6, wobei die Verbindung, die eine Thiolgruppe im Molekül aufweist und im Wesentlichen keinen Einfluss auf die Aktivität des Antikörpers ausübt, Cystein ist.

**8.** Verfahren nach einem der Ansprüche 1 bis 7, wobei der Antikörper ein F(ab')$_2$-Antikörper ist.

**9.** Verfahren nach einem der Ansprüche 1 bis 8, wobei der Antikörper ein monoklonaler Antikörper ist.

**10.** Verfahren nach Anspruch 9, wobei der monoklonale Antikörper eine Thiolgruppe in seiner variablen Region aufweist.

**11.** Verfahren nach den Ansprüchen 9 oder 10, wobei der monoklonale Antikörper einen freien Cysteinrest in seiner variablen Region aufweist.

**12.** Verfahren nach einem der Ansprüche 9 bis 11, wobei der monoklonale Antikörper die Aminosäuresequenzen, dargestellt durch SEQ ID NOs: 1, 2 und 3 in der Sequenzliste in der hypervariablen Region seiner schweren Kette, und die Aminosäuresequenzen, dargestellt durch SEQ ID NOs: 4, 5 und 6 in der Sequenzliste in der hypervariablen Region seiner leichten Kette, umfasst.

**13.** Verfahren nach einem der Ansprüche 9 bis 12, wobei der monoklonale Antikörper eine variable Region der schweren Kette, die die Aminosäuresequenz, dargestellt durch SEQ ID NO:7 in der Sequenzliste, umfasst, und eine variable Region der leichten Kette, die die Aminosäuresequenz, dargestellt durch SEQ ID NO:8 in der Sequenzliste, enthält, umfasst.

**14.** Verfahren nach einem der Ansprüche 1 bis 13, wobei der Antikörper durch die Verwendung einer in einem Serumfreien Medium kultivierten Zelle hergestellt wird.

**Revendications**

**1.** Procédé de protection d'un groupe thiol dans un anticorps ayant un résidu cystéine libre, lequel comprend l'addition d'un composé ayant une liaison disulfure dans la molécule et n'exerçant sensiblement aucune influence sur l'activité de l'anticorps, où le composé possédant une liaison disulfure dans la molécule et n'exerçant sensiblement aucune influence sur l'activité de l'anticorps est la cystine, l'homéocystine, l'acide lipoïque, le glutathion oxydé ou le disulfure de glutathion.

**2.** Procédé selon la revendication 1, lequel est destiné à inhiber une réaction de polymérisation d'anticorps par l'intermédiaire des groupes thiol.

**3.** Procédé selon la revendication 1, lequel est destiné à inhiber la modification d'un anticorps.

**4.** Procédé selon la revendication 1, lequel est destiné à inhiber une réaction d'échange d'un groupe thiol dans un anticorps avec une liaison disulfure formée dans la molécule ou entre les molécules de l'anticorps.

**5.** Procédé selon l'une quelconque des revendications 1 à 4, où le composé ayant une liaison disulfure dans la molécule et n'exerçant sensiblement aucune influence sur l'activité de l'anticorps est la cystine.

**6.** Procédé de protection d'un groupe thiol dans un anticorps ayant un résidu cystéine libre selon la revendication 1, lequel comprend l'addition du composé ayant une liaison disulfure dans la molécule et n'exerçant sensiblement aucune influence sur l'activité de l'anticorps simultanément ou distinctement d'un composé ayant un groupe thiol dans la molécule et n'exerçant sensiblement aucune influence sur l'activité de l'anticorps, où le composé ayant un groupe thiol dans la molécule et n'exerçant sensiblement aucune influence sur l'activité de l'anticorps est la cystéine, l'homocystéine, le glutathion ou l'acide dihydrolipoïque.

7. Procédé selon la revendication 6, où le composé ayant un groupe thiol dans la molécule et n'exerçant sensiblement aucune influence sur l'activité de l'anticorps est la cystéine.

8. Procédé selon l'une quelconque des revendications 1 à 7, où l'anticorps est un anticorps F(ab')$_2$.

9. Procédé selon l'une quelconque des revendications 1 à 8, où l'anticorps est un anticorps monoclonal.

10. Procédé selon la revendication 9, où l'anticorps monoclonal a un groupe thiol dans sa région variable.

11. Procédé selon la revendication 9 ou 10, où l'anticorps monoclonal a un résidu cystéine libre dans sa région variable.

12. Procédé selon l'une quelconque des revendications 9 à 11, où l'anticorps monoclonal comprend les séquences d'acides aminés représentées par SEQ ID NO: 1, 2 et 3 dans la Liste de séquences dans sa région hypervariable à chaîne lourde et les séquences d'acides aminés représentées par SEQ ID NO:4, 5 et 6 dans la Liste de séquences dans sa région hypervariable à chaîne légère.

13. Procédé selon l'une quelconque des revendications 9 à 12, où l'anticorps monoclonal comprend une région variable à chaîne lourde comprenant la séquence d'acides aminés représentée par SEQ ID NO:7 dans la Liste de séquences et une région variable à chaîne légère contenant la séquence d'acides aminés représentée par SEQ ID NO:8 dans la Liste de séquences.

14. Procédé selon l'une quelconque des revendications 1 à 13, où l'anticorps est produit par recours à une cellule cultivée dans un milieu exempt de sérum.

# FIG. 1

## FIG. 2

## FIG. 3

# FIG. 4

FIG. 5

EP 1 574 521 B1

FIG. 6

FIG. 7

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0943690 A
- WO 9013310 A
- US 6342585 B
- WO 03048357 A
- EP 526700 A
- EP 520499 A

- EP 1174126 A
- WO 8806441 A
- JP 649931 A
- JP 2002370522 A
- JP 2002370822 A

**Non-patent literature cited in the description**

- Seikagaku Jiten. Tokyo Kagaku Dojin, 182b